# EUROPEAN PATENT APPLICATION

(11) **EP 0 855 442 A2**
(43) Date of publication of application: **29.07.1998**
(21) Application number: 97309637.3
(22) Date of filing: 28.11.1997
(51) Int. Cl.: C12N 15/12, C07K 14/47, C12N 1/21

(54) **Central nervous system transcription regulator polypeptide and a DNA sequence to which a protein containing the polypeptide binds**

(30) Priority: 28.11.1996 JP 318285/96
(71) Applicant: Japan Science and Technology Corporation, Kawaguchi-shi, Saitama-ken 332 (JP)
(72) Inventor: Hotta, Yoshiki, Shizuoka (JP)
(74) Representative: Dzieglewska, Hanna Eva

(57) **Abstract**

The present invention provides a polypeptide which is common to essentially all proteins expressed by the glial cells missing (*gcm*) gene, which is a fate determination gene of animal central nervous system cells, said polypeptide having the amino acid sequence of Sequence ID No. 1 or a part, functional fragment, derivative, homologue or analogue, thereof and a DNA sequence to which proteins containing the polypeptide binds. The polypeptide and DNA sequence bound by the polypeptide facilitate study and clarification of the mechanisms by which the human nervous system is formed and opens up new ways to develop diagnostic and medical treatment regimes for cerebral functional diseases and cerebral tumours.

## Description

The present invention relates to a polypeptide common to essentially all proteins expressed from the *glial cells missing (gcm)* gene, which gene is a fate determination gene of animal central nervous system cells and a DNA sequence in animal gemonic DNA, to which the polypeptide binds. More particularly, the present invention relates to a polypeptide existing within a novel DNA-binding protein and a specific DNA sequence in genomic DNA to which this protein binds, which facilitate elucidation of the mechanisms of central nervous system (CNS) formation in animal species including humans. Furthermore, the products of the present invention allow development of diagnostic and medical treatment means for various diseases caused by structural and functional disorders of the CNS.

The central nervous system (CNS) may be viewed as a giant information processor comprising a huge number of cells of which component cells may be broadly divided into neurons and glial cells. Neurons, which are complete nerve cells including the cell bodies, axons and dendrites, are considered to play a central role in information processing by forming an electrical impulse conducting network. Glial cells, which are non-neuronal cells, provide support for the formation and functioning of the neuronal network by, for example, regulating the extracellular environment of the central nervous system and thus play an important role in repairing damage to the network. Accurate formation and effective functioning of these two types of cells is an essential prerequisite for the CNS as a whole to function normally. It is therefore very important to understand the mechanisms by which the complicated nervous system develops, to appreciate the roles and functions of the component elements and their interactions and to have a knowledge of the formation of diversity and how differential function is determined in the generation of the nervous system.

The fact that neurons and glial cells are produced from common precursor cells (stem cells) has been reported for many species. However the mechanisms by which individual stem cells are differentiated into neurons and glial cells has not as yet been clarified. While it is known that some molecules exert an effect on the determination of differentiation, it has not been shown that this effect is necessary for determining glia versus neuronal cell fate.

*Drosophila* is a model organism with a long history as an experimental tool in genetics. An important advantage of using *Drosophila* is the potential for rapidly identifying an unknown gene(s) participating in a biological process through the screening and analysis of mutants. It is now possible to determine individual cells of the central nervous system and study the development thereof in detail as a result of using cell markers for example, using various antibodies or the *lacZ* gene and the establishment of techniques for injecting dye(s) into single cells. The central nervous system of *Drosophila* is thus becoming an excellent model for research efforts on the generation and development of cells forming the nervous system and the differentiation determining mechanisms.

The CNS of *Drosophila* is composed of 30 neuroblasts per side of each segment. Neuroblasts produce both neurons and glial cells by repeating stem cell-like asymmetric divisions and about 300 neurons and about 30 glial cells are created per side of each segment before completion of the nervous system. Each of the 30 neuroblasts is formed at a predetermined time in development, at a predetermined position which permits individual identification and a name is assigned to each of them. Follow up of cells produced by specific neuroblasts permits observation of the generation of specific neurons and glial cells. In *Drosophila* therefore, the fate of each cell of the nervous system is almost entirely determined genetically.

The present inventors screened *Drosophila* mutants in an effort to identify genes participating in differentiation of the nervous system and obtained a mutant strain abnormal in the formation of glial cells. The resultant mutant was thus named *glial cells missing (gcm)* and the *gcm* gene was proposed as the basis for this mutant (Cells 82: 1025-1036, 1995). Mutation of the *gcm* gene can result in cells destined to become glia being differentiated into neurons and misexpression of the *gcm* gene in neuroblasts can cause presumptive neurons to differentiate into glia. This suggests that the *gcm* gene controls the determination of fate between neurons and glial cells.

In addition to the present inventors, three groups carried out independent analysis of the *gcm* gene and obtained results supporting the conclusion described above (Cell 82: 1013-1023, 1995; Genetics 139: 1663-1678, 1995; Development 122: 131-139, 1996; Cell 83: 671-674, 1995; Neuron 15: 1219-1222, 1995).

As discussed above, it is clear that the *gcm* gene plays an important role in the determination of the fate of nervous system cells. However, the amino acid sequence deduced from the cDNA nucleotide sequence of the *gcm* gene exhibits almost no homology with any proteins in the databases screened. Thus, the function(s) of the protein (GCM) expressed by the *gcm* gene could not be established.

In order accurately to understand the functions of a particular gene, it is generally essential to clarify the physiological activity of the expression product. It is therefore very important to understand the role of GCM in the central nervous system, with a view to elucidating the mechanisms of formation of the central nervous system as well as developing diagnostic and medical treatment techniques in relation to central nervous system diseases.

The present invention provides a polypeptide and DNA sequence which permit various and diverse industrial uses of the *gcm* gene and the expressed protein product thereof, by elucidating functions of the expressed GCM protein product of the *gcm* gene of nervous system cells.

The present invention thus provides a polypeptide common to proteins expressed by the *gcm* gene, said gene being a fate determination gene of cells of an animal central nervous system, said polypeptide having an amino acid sequence corresponding to Sequence ID No. 1 or a part, or functional fragment, derivative, homologue or analogue thereof.

Thus, the invention provides also a polypeptide corresponding to Sequence ID No. 1 or a part thereof wherein one or more amino acid residues are substituted, deleted or added. In another aspect, the present invention provides a GCM protein comprising the polypeptide as defined above.

In a further aspect, the invention provides a DNA sequence in animal genomic DNA to which a protein comprising the foregoing polypeptide binds, said DNA sequence having a nucleotide sequence corresponding to Sequence ID No. 2 or a part, a functional fragment derivative, homologue or analogue thereof. Such derivatives, homologues or analogues thereof may include nucleotides sequences from different animal genera or other species, allelic variants, degenerate and complementary sequences and sequences derived by modification of the nucleotide sequence e.g. by mutagenesis. Particularly included are sequences which hybridise under condition of high stringency to the sequence of SEQ ID No 2 or the complement thereof. Viewed from another aspect the present invention provides a vector comprising the DNA sequence as defined above and viewed from yet another aspect the present invention provides a cell transformed with such a vector and a clone of such transformed cells.

In the amino acid sequence of Sequence ID No. 1, Xaa represents an arbitrary amino acid residue and in the nucleotide sequence of Sequence ID No. 2, R represent A or G.

The polypeptide provided by the present invention and the DNA sequence to which a protein containing this polypeptide binds are useful as materials for researching the mechanisms by which the central nervous system forms and develops in various animal species and for elucidating the functions thereof. The inventions of the present application may also be used in the diagnosis and medical treatment of various diseases caused by structural or functional disturbances of the central nervous system. Thus, viewed from another aspect, the present invention provides the use of a polypeptide having an amino acid sequence corresponding to ID No. 1 or a part, or functional fragment, derivative, homologue or analogue thereof in the preparation of a therapeutic agent for the diagnosis, characterization and/or treatment of diseases which result from structural or functional disturbances of the CNS. In one particular embodiment the CNS malfunction susceptible of treatment by such use of the present invention is glial cerebral tumour.

Alternatively viewed, the present invention provides a method for controlling the *gcm* genes in stored blast glial cells by means of the protein or polypeptide of the invention, converting blast glial cells into neurons and returning the converted neurons into the brain thus allowing the recovery of brain function: the "self-brain cell transplantation technique". This aspect of the invention provides the means to control gcm-controlled (or influenced) genes e.g. in the blast glial cells *in vitro,* using the protein or polypeptide of the invention. Thus, blast glial cells isolated from a neonate may be stored and cultured or grown *in vitro,* for potential subsequent use by said neonate in adult life, when a cerebral functional disorder is developed.

The polypeptide of the invention may be isolated through hydrolysis or the like of GCM protein expressed in the central nervous system of an animal. It is also possible to prepare the polypeptide of the invention through chemical synthesis on the basis of the amino acid sequence provided by the invention. The polypeptide of the invention comprises a peptide fragment comprising any partial amino acid sequence of Sequence ID No. 1 wherein said fragment is five or more amino acid residues in length.

The DNA sequence of the invention is a DNA sequence found in genomic DNA sequences to which the GCM protein binds and may be isolated by known means or can be prepared synthetically by any known method as a DNA fragment. The DNA fragment may be used as a probe for identifying a gene(s) to which the GCM protein binds or alternatively, may be used as a primer for PCR-amplification of such a gene and as such these form further aspects of the present invention. Other uses of such a nucleic acid sequence as are known to the skilled person are also within the scope of the present invention.

The present invention will now be illustrated by the following non-limiting examples in which:
Fig. 1A is a schematic diagram of GCM fusion protein used in the gel shift assay; and Fig. 1B is a representation of the result of the gel shift assay after protein electrophoresis;
Fig. 2A illustrates the matching ratio of the GCM binding sequence based on sequence alignment; and Fig. 2B is a representation showing the result of an antagonistic assay after protein electrophoresis;
Fig. 3A illustrates GCM binding sites in the upstream region of the *repo* gene; and Fig. 3B shows the DNA sequence at these sites;
Fig. 4 illustrates, aligned GCM amino acid sequences of human, mouse and *Drosophila* species and illustrates conserved regions; and
Fig. 5 illustrates partial amino acid sequences of GCM from mouse and *Drosophila.*

### Example 1

### (1) DNA binding properties of GCM

The *gcm* gene of *Drosophila* encodes a 504 amino acid protein. It is clear from searching for amino acid sequences in the database, that this protein has no homologous sequence motif characterized to date, other than a nuclear localization signal. The GCM protein is a novel nuclear protein. The protein brings about changes in the level of expression and other changes in many genes and functions, at least in part, as a transcription regulating factor, controlling the expression of its target genes. The DNA binding properties of GCM were investigated.

The upstream region of the *repo* gene of *Drosophila* was selected as a target for GCM binding. The homeobox gene *repo* is expressed in virtually all glial cells from an early stage onwards and this expression is strongly dependent on *gcm.* If therefore GCM is a transcription regulating factor, the *repo* gene should be a suitable candidate target gene.

### a) Procedures

Fusion proteins of various regions of GCM and maltose-binding protein were prepared as expression products of *Escherichia coli* and the capacity of the fusion proteins to bind with various DNA fragments prepared from the genomic upstream region of the *repo* gene was investigated by the gel shift assay.

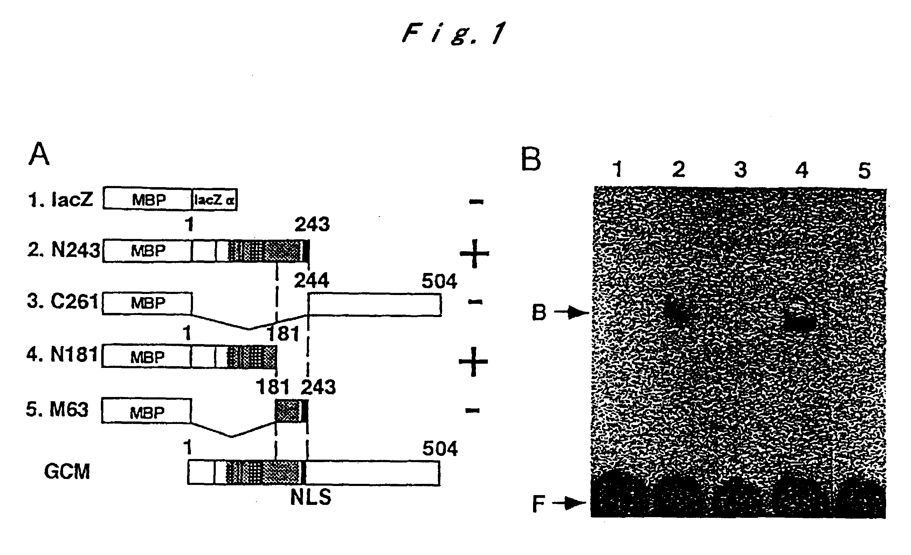

Production of fusion proteins: Fusion proteins were prepared using a protein and purification system (made by New England Biolabs). Various segments of the *gcm* gene were amplified by the PCR method with pfu polymerase (TOYOBO), and inserted in a pMAL^{™}-cz vector, which was then introduced into *Escherichia coli* BL21 (DE3)pLysS. Fusion proteins and MBP-lacZp, which was used as a control for the gel shift assay, were expressed in the transformed *Escherichia coli* and then sonicated, extracted and affinity-purified using amylose resin. The structure of each fusion protein is shown in Fig. 1A: 1 is the control protein comprising no CMG sequence information ; 2-5 are fusion proteins comprising fragments of CMG as shown by the hatched areas; and 6 is a schematic view of the GCM protein. Numbers attached to each diagram represent positions of the amino acid residues of GCM.

Preparation of DNA fragments from the upstream region of the *repo* gene: Clones containing the *repo* gene were isolated from a *Drosophila* genomic library. After confirming the nucleotide sequence thereof, the *repo* gene upstream region of about 7 kb was excised with multiple restriction enzymes to prepare 60-460 bp DNA fragments.

Gel shift assay: aproximately 200ng of each fusion protein incubated in accordance with a known method (Cell 64: 439-446, 1991; EMBO J. 10:2965-2973, 1991) with 100 bp of ³²P-labeled DNA at 25°C for 30 minutes and then subjected to electrophoresis at 4°C with polyacrylamide.

### b) Results

The results of the gel shift assay are shown in the representation of the electrophoresis gel of Fig. 1B. The lane numbers correspond to proteins of which the structures are shown in Fig. 1A. The fusion proteins 2 (N243) and 4 (N181) bound to the DNA fragments in the *repo* gene upstream region (arrow B in the drawing) confirming that GCM is a DNA-binding protein and at the same time suggests that the DNA-binding sequence is present in the region of up to the 181st amino acid at the amino terminal of GCM. Since the amino acid sequence of up to the 181st amino acid does not exhibit homology with any known proteins, it is a novel DNA-binding domain.

### 2) DNA sequence to which the DNA binding domain of GCM binds

It was investigated whether or not the DNA-binding domain of GCM binds by recognizing a specific or consensus sequence.

### a) Procedures

Gel shift assay: In accordance with a known method (Science 250: 1104-1110, 1990; Cell 64: 459-470, Science 257: 1951-1955, 1192; Cell 68: 283-302, 1992) probes were prepared from oligonucleotides resulting from insertion of 15 bp of random sequence into the nucleotide sequences of Sequence ID Nos. 3 and 4 respectively and a gel shift assay was carried out in the same manner as above by reacting the disrupted nucleotide sequences with fusion protein N243. Oligonucleotides binding with the fusion protein were isolated and PCR-amplified with the sequences of Sequence ID Nos. 3 and 4 as primers and the resultant PCR products and fusion protein were examined by gel shift assay. This cycle was repeated three times and the sequences of the finally obtained PCR products were determined.

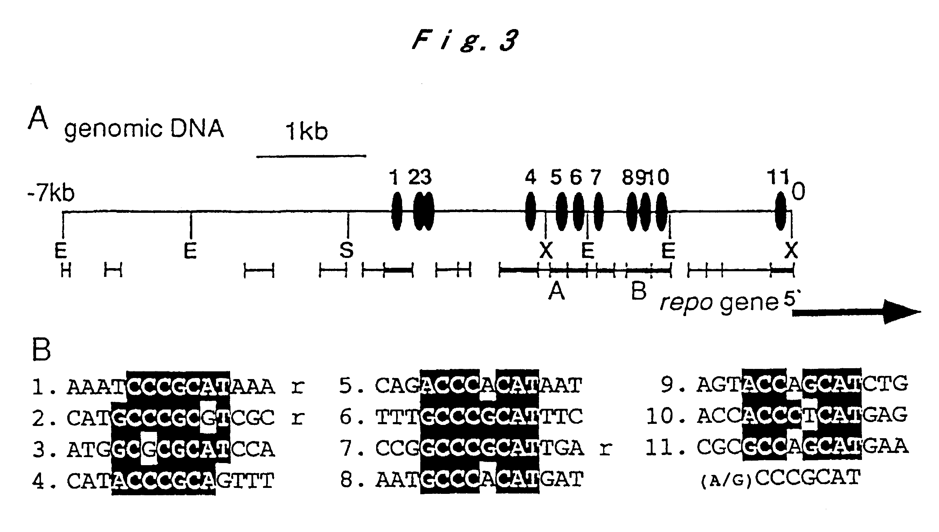

Competition assay: Competitive oligonucleotide exhibiting the nucleotide sequences of Sequence ID Nos. 5 and 6 were previously caused to react with fusion protein N243 and then with N243 with a labelled 200 bp DNA fragment (Fig. 3A and 3B) to carry out a gel shift assay in the same manner as above.

### b) Results

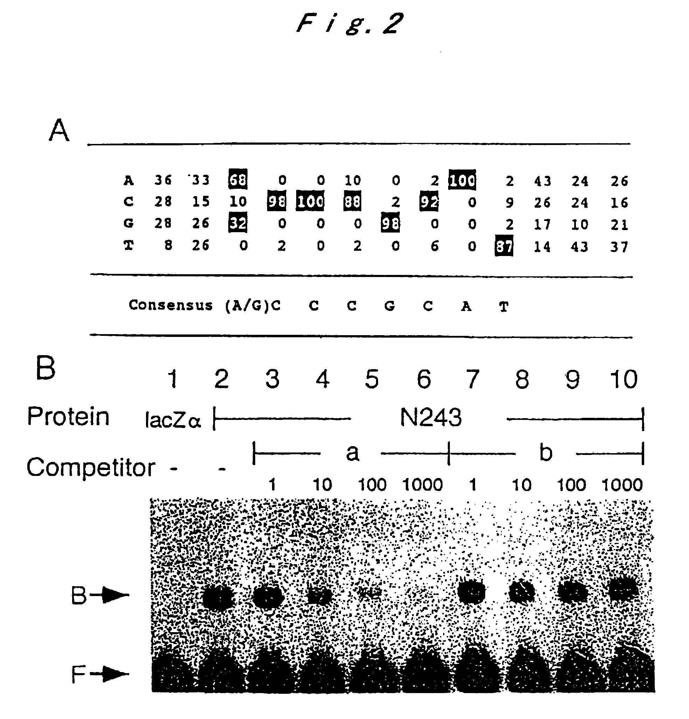

Forty-eight clones of oligonucleotides were obtained through the gel shift assay of oligonucleotides and fusion protein and three repetitions of PCR-amplification of the protein bound oligonucleotides. Among them, 38 clones had common nucleotide sequences shown by Sequence ID No. 2. Eleven clones had sequences that contained a single base mismatch, and the remaining three clones had only two base mismatches. The sequence alignment of each base is as shown in Fig. 2A which shows a high alignment within a range of 87 to 100%. This result strongly suggests that the GCM protein specifically recognizes the nucleotide sequence of Sequence ID No. 2 upon binding to DNA.

The Sequence ID No. 2 was further confirmed as a GCM-binding sequence by the competition assay (Fig. 2B) More specifically, when reacting the competitive oligonucleotide of Sequence ID No. 5 including the sequence of Sequence ID No. 2 (Fig. 2B-a) with GCM binding property of the probe decreases according to the concentration thereof (1, 10, 100 and 1,000 times), whereas with Sequence ID No. 6 which does not include the sequence of Sequence ID No. 2 no competition with the probe was observed.

### (3) GCM binding sites in the upstream region of the repo gene. The GCM binding site was searched for in the region upstream of the repo gene.

Gel shift assays were carried out by the use of 21 non-overlapping DNA fragments (Fig. 3A: bottom horizontal bar) and the fusion protein N243 to identify the DNA fragments to which the protein binds.

N243 bound to eight DNA fragments (Fig. 3A: bottom thick horizontal bar). C261 on the other hand did not bind to any of the DNA fragments.

The DNA sequence of the 7 kb upstream region was then investigated. It was found that eleven GCM-binding sequences (Sequence ID No. 2) existed within 4 kb upstream region, while no binding sequence was present in the -4 to -7 kb upstream region. The sequence of these 11 sites are as shown in Fig. 3B: those having seven out of eight matching bases were counted as GCM-binding sites. All of the eight foregoing DNA fragments binding to N243 contained one or more GMC-binding sequences.

The fact that as many as eleven sites of GCM-binding sequence clusters are present in the upstream region of the *repo* gene suggests that GCM directly controls expression of the *repo* gene as a transcription regulating factor.

### (4) GMC binding consensus sequence in different species of animal.

To determine whether the DNA binding domain is conserved throughout evolution, mammalian homologues were compared with *Drosophila* GCM.

A human gene (hGCMa) was derived from the EST database. Because the sequences in this database were not complete, a complete code sequence list was prepared by the 5'-RACE (rapid amplification of cDNA ends) and 3'-RACE methods. Mouse genes (nGCMa and mGCMb) were isolated by using the conserved region between GCM and hGCMa. More specifically, mGCMa was prepared from mouse placenta poly(A)+RNA by the reverse transcriptase PCR(RT-PCR) method, and mGCMb from mouse brain poly(A)+RNA by the RT-PCR method.

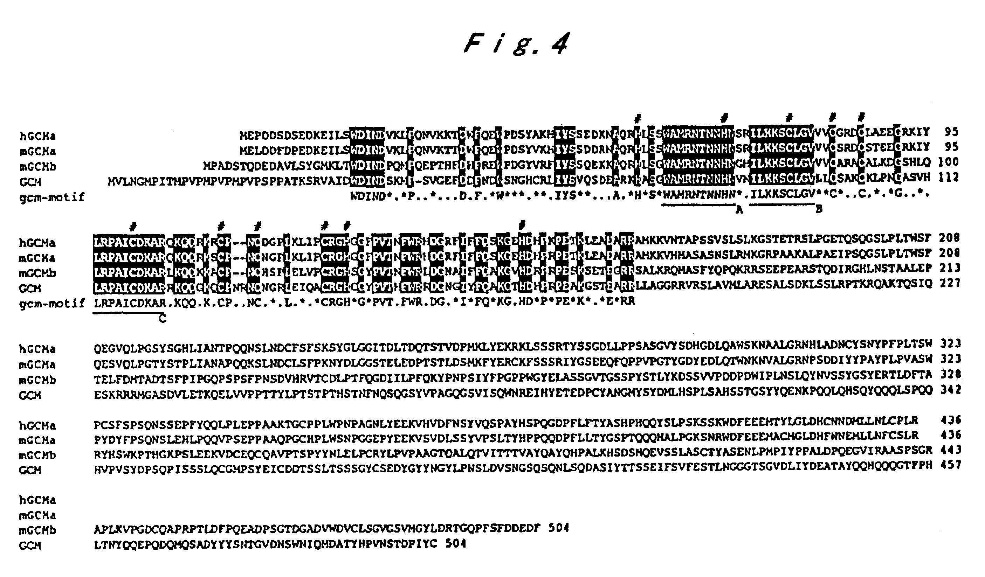

Comparison of the amino acid sequences of the protein deduced from these human and mouse genes and the amino acid sequence of GCM, revealed strong conservation of the highly basic amino-terminal third of the position as shown in Fig. 4. A conserved motif can be unambiguously defined from these comparisons, the defined motif being named the "gcm-motif". Further, this motif corresponds also to the DNA-binding domain of GCM (1-181 amino acid residues). Comparison of the individual sequences reveals the presence of three absolutely conserved stretches of nine to ten amino acid residues (A, B and C in Fig. 4) and seven conserved cysteine and four conserved histidine residues. In contrast to the highly conserved gcm-motif at the amino-terminal regions, the carboxy-terminal regions have essentially no similarity to each other nor to any known proteins.

Gel shift assays and competition assays were carried out in the same manner as above by using proteins obtained by expressing DNA fragments of up to the 171-st amino acid residue of hGCMa. Results similar to those of GCM were generated. These results also suggest that the gcm-motif is a sequence containing a specific DNA-binding domain.

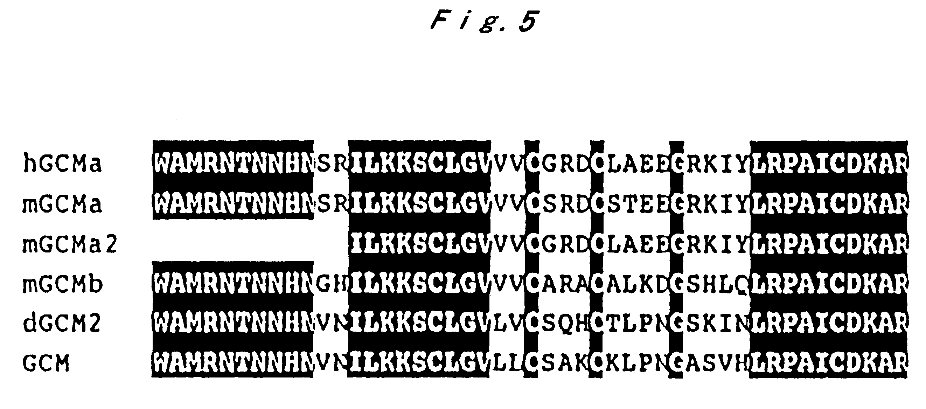

Furthermore, presence of the gcm-motif was also confirmed in the RT-PCR product the (mGCMa2) using mouse brain poly(A)+RNA as a template and the PCR product (dGCM2) using the *Drosophila* genomic DNA as template (Fig. 5) suggests that the gcm-motif is conserved in many animal species and that the proteins containing this sequence form a novel family of DNA-binding proteins.

According to the present invention, as described above in detail, the glial cells missing *(gcm)* gene of the central nervous system of various animals commonly exists and is expressed as GMC proteins. There is provided a polypeptide having a specific amino acid sequence which functions as a DNA-binding domain of the GCM protein and a DNA sequence to which the GCM protein binds. The present invention contributes immeasurably to the process of clarifying the molecular mechanisms by which the nervous system of humans and other animals forms and opens up new ways to develop diagnostic and medical treatment regimes for cerebral functional diseases and cerebral tumours using the *gcm* genes, the expressed proteins thereof, and the conserved peptide and the GCM regulated genes and gene products.

SEQUENCE LISTING

(1) GENERAL INFORMATION:

(i) APPLICANT:
(A) NAME: Japan Science and Technology Corporation
(B) STREET: 4-1-8 Hon-cho, Kawaguchi-shi
(C) CITY: Saitama
(E) COUNTRY: Japan
(F) POSTAL CODE (ZIP):

(ii) TITLE OF INVENTION: Polypeptide and a DNA sequence to which a
protein containing the polypeptide binds

(iii) NUMBER OF SEQUENCES: 6

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97309637.3

(2) INFORMATION FOR SEQ ID NO: 1:

(i) SEQUENCE CHARACTERISTICS:
(A) LENGTH: 154 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 1:

Trp Asp Ile Asn Asp Xaa Xaa Xaa Pro Xaa Xaa Xaa Xaa Xaa Xaa Asp
1               5                   10                  15

Xaa Phe Xaa Xaa Trp Xaa Xaa Xaa Xaa Xaa Xaa Xaa Ile Tyr Ser Xaa
            20                  25                  30

Xaa Xaa Xaa Xaa Ala Xaa Xaa His Xaa Ser Xaa Trp Ala Met Arg Asn
        35                  40                  45

Thr Asn Asn His Asn Xaa Xaa Ile Leu Lys Lys Ser Cys Leu Gly Val
    50                  55                  60

Xaa Xaa Cys Xaa Xaa Xaa Cys Xaa Xaa Xaa Xaa Gly Xaa Xaa Xaa Xaa
65                  70                  75                  80

Leu Arg Pro Ala Ile Cys Asp Lys Ala Arg Xaa Lys Gln Gln Xaa Lys
                85                  90                  95

Xaa Cys Pro Xaa Xaa Asn Cys Xaa Xaa Xaa Leu Xaa Xaa Xaa Xaa Cys
            100                 105                 110

Arg Gly His Xaa Gly Xaa Pro Val Thr Xaa Phe Trp Arg Xaa Asp Gly
        115                 120                 125

Xaa Xaa Ile Xaa Phe Gln Xaa Lys Gly Xaa His Asp Xaa Pro Xaa Pro
    130                 135                 140

Glu Xaa Lys Xaa Xaa Xaa Glu Xaa Arg Arg
145                 150

(2) INFORMATION FOR SEQ ID NO: 2:

(i) SEQUENCE CHARACTERISTICS:
(A) LENGTH: 8 base pairs
(B) TYPE: nucleic acid
(C) STRANDEDNESS: double
(D) TOPOLOGY: linear

(ii) MOLECULE TYPE: DNA (genomic)

(xi) SEQUENCE DESCRIPTION: SEQ ID NO: 2:

RCCCGCAT                                                    8

(2) INFORMATION FOR SEQ ID NO: 3:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 3:

TGTGTGGAAT TGTGAGCGGA                                       20

(2) INFORMATION FOR SEQ ID NO: 4:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 4:

GGTTTTCCCA GTCACGACG 19

(2) INFORMATION FOR SEQ ID NO: 5:

(i) SEQUENCE CHARACTERISTICS:
(A) LENGTH: 54 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 5:

TGTGTGGAAT TGTGAGCGGA CCTACCCGCA TTACGGGTTT
TCCCAGTCAC GACG 54

(2) INFORMATION FOR SEQ ID NO: 6:

(i) SEQUENCE CHARACTERISTICS:
(A) LENGTH: 54 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 6:

TGTGTGGAAT TGTGAGCGGA TATACTAATT TGTTAGGTTT
TCCCAGTCAC GACG 54

SEQUENCE LISTING

Sequence ID No. 1
Length: 154
Type: Amino acid
Topology: Linear
Molecule Type: Protein
Features of sequence: Part of sequence common to GCM proteins
of various species.
Sequence:
Trp Asp Ile Asn Asp Xaa Xaa Xaa Pro Xaa Xaa Xaa Xaa Xaa Xaa
1               5                   10                  15
Asp Xaa Phe Xaa Xaa Trp Xaa Xaa Xaa Xaa Xaa Xaa Xaa Ile Tyr
                20                  25                  30
Ser Xaa Xaa Xaa Xaa Xaa Ala Xaa Xaa His Xaa Ser Xaa Trp Ala
                35                  40                  45
Met Arg Asn Thr Asn Asn His Asn Xaa Xaa Ile Leu Lys Lys Ser
                50                  55                  60
Cys Leu Gly Val Xaa Xaa Cys Xaa Xaa Xaa Cys Xaa Xaa Xaa Xaa
                65                  70                  75
Gly Xaa Xaa Xaa Xaa Leu Arg Pro Ala Ile Cys Asp Lys Ala Arg
                80                  85                  90
Xaa Lys Gln Gln Xaa Lys Xaa Cys Pro Xaa Xaa Asn Cys Xaa Xaa
                95                  100                 105
Xaa Leu Xaa Xaa Xaa Xaa Cys Arg Gly His Xaa Gly Xaa Pro Val
                110                 115                 120
Thr Xaa Phe Trp Arg Xaa Asp Gly Xaa Xaa Ile Xaa Phe Gln Xaa
                125                 130                 135
Lys Gly Xaa His Asp Xaa Pro Xaa Pro Glu Xaa Lys Xaa Xaa Xaa
                140                 145                 150

Glu Xaa Arg Arg
154

Sequence ID No. 2
Length: 8
Type: Nucleic acid
Strandedness: Double
Topology: Linear
Molecule Type: Genomic DNA
Features of sequence: DNA sequence to which GCM protein binds
containing amino acid sequence of
sequence No. 1
Sequence:
RCCCGCAT 8

Sequence ID No. 3
Length: 20
Type: Nucleic acid
Strandedness: Single
Topology: Linear
Molecule Type: Synthetic DNA
Sequence:
TGTGTGGAAT TGTGAGCGGA 20

Sequence ID No. 4
Length: 19
Type: Nucleic acid
Strandedness: Single
Topology: Linear
Molecule Type: Synthetic DNA

Sequence:

GGTTTTCCCA GTCACGACG 19

Sequence ID No. 5

Length: 54

Type: Nucleic acid

Strandedness: Single

Topology: Linear

Molecule Type: Synthetic DNA

Sequence:

TGTGTGGAAT TGTGAGCGGA CCTACCCGCA TTACGGGTTT TCCCAGTCAC 50

GACG 54

Sequence Id No. 6

Length: 54

Type: Nucleic acid

Strandedness: Single

Topology: Linear

Molecule Type: Synthetic DNA

Sequence:

TGTGTGGAAT TGTGAGCGGA TATACTAATT TGTTAGGTTT TCCCAGTCAC 50

GACG 54

## Claims

1. A polypeptide common to proteins expressed by the glial cells missing *(gcm)* gene, said polypeptide having the amino acid sequence of Sequence ID No. 1, or a part, functional fragment, derivative, homologue or analogue thereof.

2. The polypeptide of claim 1 wherein one or more amino acid residues are substituted, deleted or added.

3. A protein comprising a polypeptide motif as defined in either of claims 1 or 2.

4. A DNA sequence in animal genomic DNA to which the polypeptide of claims 1 or 2 or the protein of claim 3 binds, said DNA sequence having the nucleotide sequence of Sequence ID No. 2, or a part, a functional fragment derivative, homologue or analogue thereof.

5. A DNA vector comprising the DNA sequence of claim 4.

6. A cell transformed with the vector of claim 5.

7. A DNA or RNA oligonucleotide comprising a nucleotide sequence corresponding to, complementary or being a transcript of the nucleotide sequence of Sequence ID No. 2 or a part, a functional fragment derivative, homologue or analogue thereof.

8. Use of a polypeptide as defined in claims 1 to 2, a protein as defined in claim 3 or the DNA sequence defined in claim 4 in the preparation of a therapeutic agent for the diagnosis, characterization and/or treatment of diseases which results from a structural or functional disturbance of the CNS.

9. Use as claimed in claim 8 wherein said CNS malfunction is glial cerebral tumour.

10. Use as claimed in claim 8 wherein stored blast glial cells are converted into neurons suitable for transplantation into the CNS of a human or other animal.
